# EUROPEAN PATENT APPLICATION

(11) **EP 1 350 530 A1**
(43) Date of publication of application: **08.10.2003**
(21) Application number: 03007350.6
(22) Date of filing: 01.04.2003
(51) Int. Cl.: A61M 5/32

(54) **Safety shield assembly**

(30) Priority: 02.04.2002 US 115120; 24.05.2002 US 156611
(71) Applicant: Becton Dickinson and Company, Franklin Lakes, New Jersey 07417-1880 (US)
(72) Inventor: Crawford, Jamieson William Maclean, New York, New York 10025 (US)
(74) Representative: Müller-Boré & Partner Patentanwälte

(57) **Abstract**

The present invention is a safety shield assembly having a shield and a collar for connecting the shield to a fluid handling device whereby the shield may be pivoted with respect to the collar. Preferably, the safety shield assembly may be used with a needle assembly, an intravenous infusion set a syringe, a catheter or other fluid handling devices or assemblies that contain piercing elements.

## Description

### 1. Field of the Invention

The present invention relates to a shield for a needle and more particularly to a safety shield assembly that may be used in conjunction with a syringe assembly, a hypodermic needle, a needle assembly, a needle assembly with a needle holder, a blood collection needle, a blood collection set, an intravenous infusion set or other fluid handing devices or assemblies that contain piercing elements.

### 2. Background of the Invention

Disposable medical devices having piercing elements for administering a medication or withdrawing a fluid, such as hypodermic needles, blood collecting needles, fluid handling needles and assemblies thereof, require safe and convenient handling. The piercing elements include, for example, pointed needle cannula or blunt ended cannula.

Safe and convenient handling of disposable medical devices is recognized by those in the medical arts so as to minimize exposure to blood borne pathogens. Safe and convenient handling of disposable medical devices results in the disposal of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies. For example, some devices employ very short thin needle cannulas. A shield designed to lock near the distal end of one needle cannula might not engage a much shorter needle cannula. Additionally, a shield designed to lock with a wider gauge needle cannula might be more likely to generate a spray upon engaging a much narrower needle cannula. Furthermore, it may be desirable to reduce the force required to effect shielding without reducing the audible and tactile indications of complete shielding.

Therefore, there exists a need for a safety shield assembly: (i) that is manufactured easily; (ii) that is applicable to many devices; (iii) that is simple to use with one hand; (iv) that can be disposed of safely; (v) that does not interfere with normal practices of needle use; (vi) that has tactile features whereby the user may be deterred from contacting the needle, the user may easily orient the needle with the patient and easily actuate and engage the shield assembly; (vii) that has visual features whereby the user may be deterred from contacting the needle, the user may easily orient the needle with the patient and easily actuate and engage the shield assembly; (viii) that is not bulky; (ix) that includes means for minimizing exposure to the user of residual fluid leaking from the needle; and (x) provides minimal exposure to the user because the needle shield is immediately initiated by the user after the needle is withdrawn from the patient's vein.

### 3. Summary of the Invention

The present invention is a safety shield assembly that comprises: a shield; means for connecting the shield to a fluid handling device that contains a piercing element, such as needle; means for pivoting the shield away from the needle; and means for securely covering and/or containing the needle within the shield.

Preferably, the shield comprises a rearward end, a forward end, a slot or longitudinal opening for housing the used needle in the forward end, means for securing the needle in the slot, means for guiding the needle into the slot, means for connecting the shield and the fluid handling device, means for guiding the user's fingers to move the shield into various positions, and means for retaining the shield securely over the used needle.

Desirably, the means for connecting the shield to the fluid handling device is a collar. Preferably, the shield is connected movably to a collar which is connected to a fluid handling device.

Preferably, the shield is connected to the collar by a hanger bar that engages with a hook arm on the collar so that the shield may be pivoted with respect to the collar into several positions. It is within the purview of the present invention to include any structure for connecting the shield to the collar so that the shield may be pivoted with respect to the collar. These structures include known mechanical hinges and various linkages, living hinges, or combinations of hinges and linkages.

Most preferably, the shield is connected to the collar by an interference fit between the hanger bar and the hook bar. Therefore, the shield always is oriented in a stable position and will not move forward or backwards unless movement of the shield relative to the hanger bar and the hook bar is initiated by the user.

Alternatively, the shield and collar may be a unitary one-piece structure. The one-piece structure may be obtained by many methods, including molding the shield and the collar as a one-piece unit, thereby eliminating the separate shield and collar during the manufacturing assembly process.

The assembly of the present invention may further comprise tactile and visual means for deterring the user from contacting the needle, providing easy orientation of the needle with the patient and providing the user with a guide for actuation and engagement with the shield.

The assembly of the present invention may further comprise means for minimizing exposure by the user to residual fluid leaking from a used needle. For example, a polymer material, such as a gel, may be located in the shield.

Most desirably, the assembly of the present invention is such that the cooperating parts of the assembly provide the means for the shield to move into a forward position over the needle. Thus, by simple movement of the shield into a forward position over the used needle, the assembly is ready for subsequent disposal. Therefore, the safety shield assembly of the present invention provides minimal exposure of the user to a needle because the shielding is initiated by the user immediately after the needle is withdrawn from the patient's vein.

Desirably, the assembly of the present invention may be used with a syringe assembly, a hypodermic needle, a needle assembly, a needle assembly with a needle holder, a blood collection set, an intravenous infusion set or other fluid handling devices. Preferably, the assembly of the present invention is used with a needle assembly comprising a needle and a hub. Preferably the needle is a conventional double ended needle.

Most preferably, the present invention is used with a needle assembly comprising a hub and a needle connected to the hub whereby the needle comprises a non-patient end and an intravenous end. The collar of the present invention may comprise a hook arm and the shield may be connected movably to the hook arm. Thus the shield may be positioned with respect to the collar and moved easily into several positions.

Preferably, the collar is fitted non-rotatably with the hub of the needle assembly. Additionally, the collar includes cooperating means that mate with reciprocal means on the shield to provide a clear audible and tactile indication of shielding.

The shield preferably includes at least one cannula finger lock for locked engagement with the cannula when the shield is in the second position around the needle cannula. The cannula finger lock preferably projects obliquely from one sidewall of the shield angularly toward the opposed sidewall and the top wall of the shield. The cannula finger lock is dimensioned, disposed and aligned to contact the needle cannula when the shield approaches the second position. Contact between the cannula and the cannula finger lock will cause the cannula finger lock to resiliently deflect toward the sidewall from which the cannula finger lock extends. Sufficient rotation of the shield will cause the needle cannula to pass the cannula finger lock. As a result, the cannula finger lock will resiliently return to or toward its undeflected condition for securely trapping the needle cannula in the shield.

The shield also may include at least one cannula shelf lock. The cannula shelf lock projects substantially rigidly from a sidewall of the shield. The cannula shelf lock may be a generally triangular panel with a lower edge that is inclined closer to the top wall of the shield at further distances from the sidewall on which the shelf lock is disposed. The shelf lock may further include a top edge that extends substantially parallel to the axis of rotation of the shield and/or substantially parallel to the top wall of the shield. The top edge of the shelf lock may include a recess or groove approximately symmetrically between the sidewalls of the shield for trapping the needle cannula. The cannula shelf lock functions differently from the cannula finger lock. In particular, the cannula finger lock is dimensioned and aligned to deflect in response to engagement with the needle cannula. The cannula shelf lock, on the other hand, is dimensioned and aligned to generate deflection of the needle cannula. Thus, the cannula shelf lock will cause the needle cannula to deflect transversely a sufficient distance for the needle cannula to clear the shelf lock. After sufficient rotation, the needle cannula will clear the shelf lock and resiliently return toward an undeflected condition. Thus, the cannula shelf lock will substantially prevent a re-exposure of the used needle cannula.

Preferably, the collar is fitted with the hub of the needle assembly so that the collar cannot rotate around the hub.

Alternatively, the collar and hub may be a unitary one-piece structure. The one piece structure may be accomplished by many methods including molding the collar and the hub as a one-piece unit thereby eliminating the need to separately assemble the collar to the hub during the manufacturing process.

Most preferably, the collar is fitted with the hub of the needle assembly so that the bevel surface or bevel up surface of the intravenous or distal end of the needle faces the same side of the collar when the shield is in the open position. Alignment of the collar, hub, shield and needle with the bevel surface up makes it easier to insert the needle into the patient without manipulating the assembly. The orientation of the intravenous end of the needle with the bevel up assures the user that the needle is properly oriented for use and does not require any manipulation before use. Most notably, the orientation of the shield provides a visual indication to the user of the orientation of the bevel surface of the needle.

Preferably, the shield is capable of pivoting from a first position, where the intravenous end of the needle is exposed and bevel up, to an intermediate position where the needle is partially covered, to a second position where the needle is contained by the shield.

Alternatively, it is within the purview of the present invention that the shield, collar and hub is a unitary one-piece structure. The one-piece structure may be accomplished by many methods including molding the shield, collar and hub as a one- piece unit thereby eliminating the need to separately assemble the shield, collar and hub during the manufacturing process.

It is an advantage of the present invention that the shield covering the used intravenous end of the needle provides easy containment of the used needle. A further advantage of the shield is that it will only move upon initiation by the user.

The assembly of the present invention when used with a fluid handling device is also easily disposable when removed from a conventional needle holder, or other such device.

A notable attribute of the present invention is that it is easily adaptable with many devices. For example, the invention is usable with syringe assemblies, hypodermic needles, needle holders, blood collection needles, blood collection sets, intravenous infusion sets such as catheters or other fluid handling devices or assemblies that contain piercing elements.

Another notable attribute of the present invention is that the tactile and visual features deter the user from touching the needle, allow the user to easily orient the needle with the patient and guide the user to actuate and engage the shield of the assembly.

### 4. Brief Description of the Drawings

FIG. 1 is a perspective view of the safety shield assembly of the present invention as connected to a needle assembly and related packaging features.

FIG. 2 is a perspective view of the unassembled pieces of FIG. 1.

FIG. 3 is a bottom view of the shield as shown in FIG. 2.

FIG. 4 is a cross sectional view of the collar as shown in of FIG. 2 taken along lines 4-4 thereof.

FIG. 5 is a cross sectional view of the needle hub as shown in FIG. 2 taken along lines 5-5 thereof.

FIG. 6 is a cross sectional view of the shield of FIG. 2 taken along lines 6-6 thereof.

FIGS. 7-12 illustrate the use of the safety shield assembly with the needle assembly of FIG. 1 with a conventional needle holder.

FIG. 13 is a cross sectional view of the assemblies in use with a conventional needle holder as shown in FIG. 12 taken along lines 13-13 thereof.

FIG. 14 is a cross-sectional view of the assemblies of FIG. 13 taken along lines 14-14 thereof.

FIG. 15 is a bottom view of the assemblies as shown in FIG. 11.

FIG. 16 illustrates an additional embodiment of the present invention, whereby a gel material is located in the shield as shown in a bottom view of the assemblies of FIG. 11.

FIG. 17 is a bottom view similar to FIG. 3A, but showing an alternate shield.

FIG. 18 is a cross-sectional view similar to FIG. 6, but showing the alternate shield of FIG. 17.

FIG. 19 is a cross-sectional view similar to FIG. 13, but showing the alternate shield of FIGS. 16 and 17.

FIG. 20A is a cross-sectional view taken along line 20A-20A of FIG. 19.

FIG. 20B is a cross-sectional view taken along line 20B-20B of FIG. 19.

FIG. 21 is a bottom plan view of the assembly as shown in FIG. 19.

FIG. 22 is a perspective view of an additional embodiment of the present invention in use with a blood collection set.

FIG. 23 is a perspective view of an additional embodiment of the present invention in use with a syringe.

FIG. 24 is a perspective view of an additional embodiment of the present invention in use with a catheter.

### 5. Detailed Description of the Invention

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

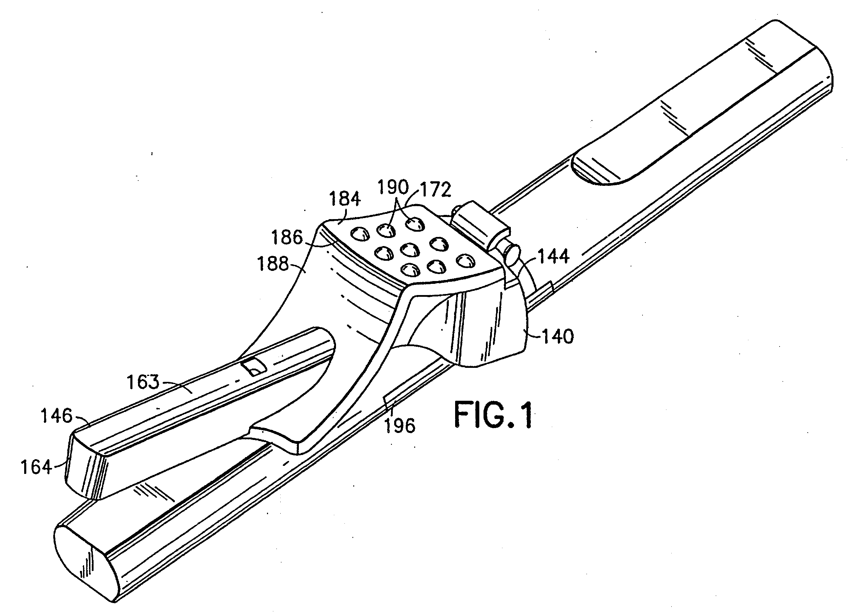

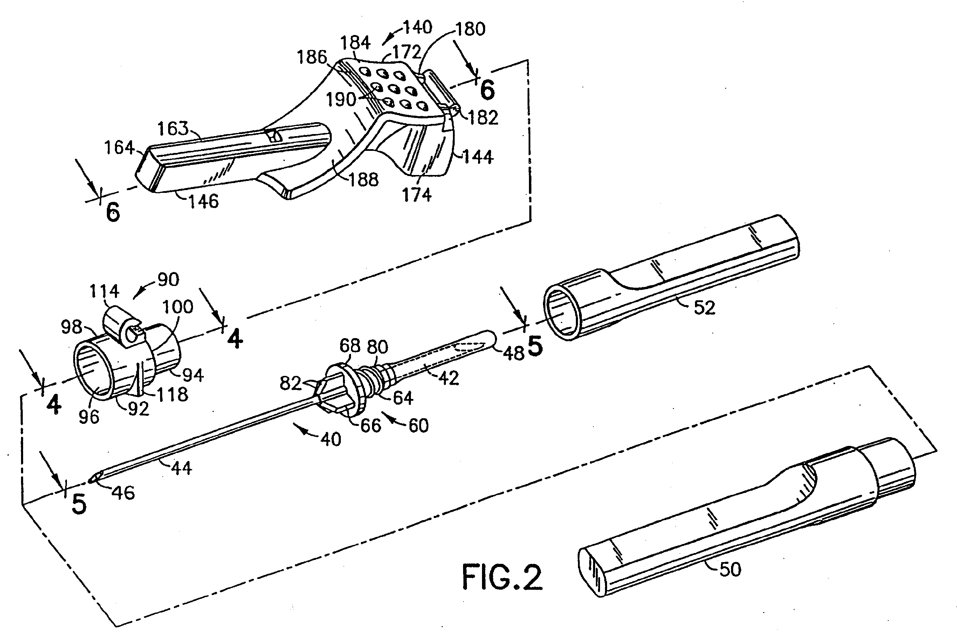

Referring to the drawings in which like reference characters refer to like parts throughout the several views thereof, FIGS. 1 and 2 illustrate a needle assembly with the safety shield assembly of the present invention and the related packaging features. The needle assembly includes a needle **40**, a hub **60**, packaging features to cover the needle and a label. The safety shield assembly includes a collar **90** and a shield **140**.

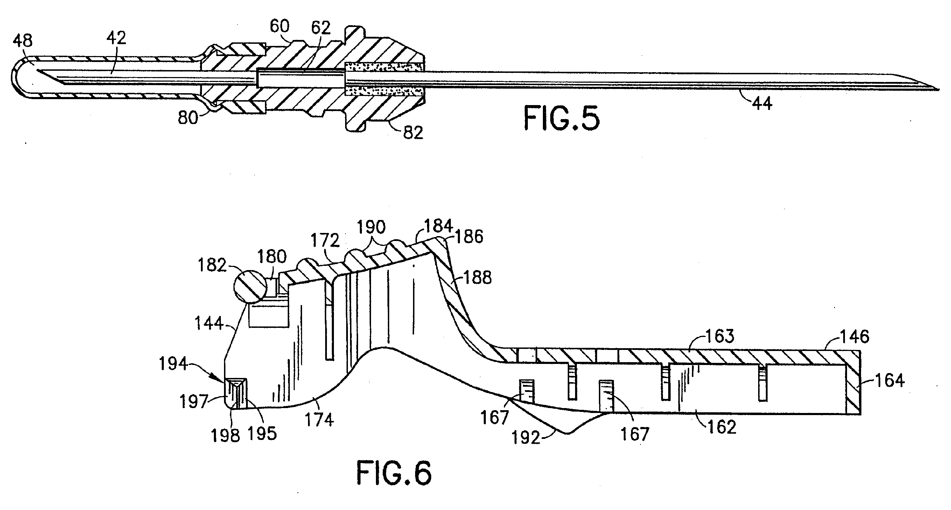

As shown in FIG. 2 and 5, needle **40** includes a non-patient end **42**, an intravenous end **44** and a passageway **46** extending between the non-patient end and the intravenous end. An elastomeric sleeve **48** covers the non-patient end. A first rigid sleeve **50** covers the intravenous end and a second rigid sleeve **52** covers the non-patient end and the elastomeric sleeve. As shown in FIG. 1, a label **196** may also be applied to the finally assembled parts.

As shown in FIGS. 2 and 5, hub **60** includes a threaded end **64**, a ribbed end **66** and passageway **62** extending between the threaded end and the ribbed end. Threaded end **64** and ribbed end **66** are separated by flange **68**. Non-patient end **42** of needle **40** extends from threaded end **64** and intravenous end **44** of needle **40** extends from ribbed end **66**. Preferably, threaded end **64** comprises male threads **80** for mounting the hub on a conventional needle holder and ribbed end **66** comprises male ribs **82** for connecting the hub and collar **90**.

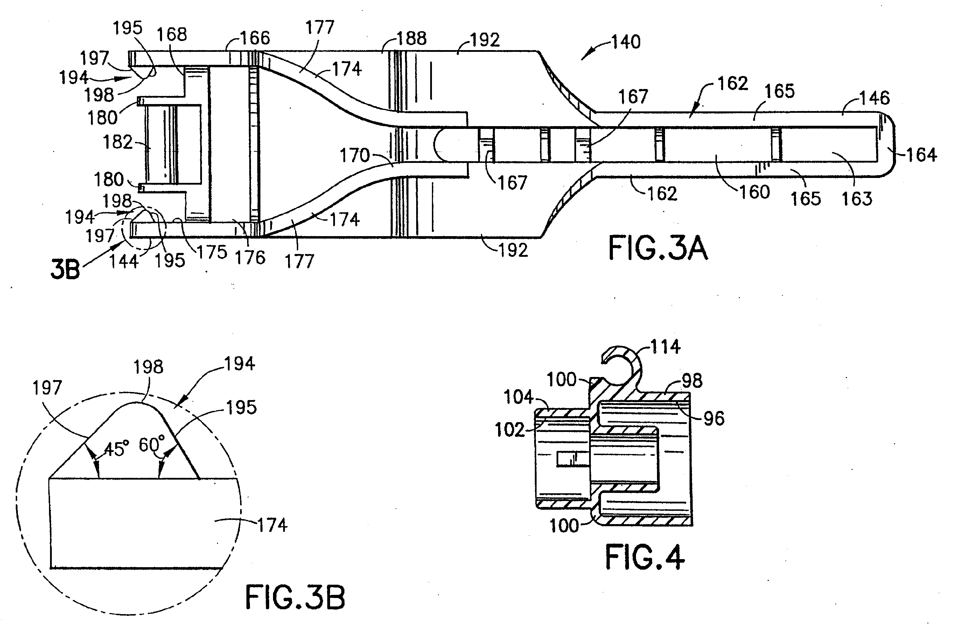

As shown in FIGS. 2 and 4, collar **90** includes a forward skirt **92** and a rearward skirt **94.** Forward skirt **92** is cylindrical and comprises an inner circumferential surface **96** and an outer circumferential surface **98**. Forward skirt **92** mates with rearward skirt **94** at a shoulder **100**. Rearward skirt **94** is cylindrical and comprises an inner circumferential surface **102** and an outer circumferential surface **104** and extends from shoulder **100** opposite of forward skirt **92**. The inner diameter of forward skirt **92** is larger than the inner diameter of rearward skirt **94**. Alternatively, the inner diameters for collar **90** can be equal. A hook **114** extends from outer circumferential surface **98** of forward skirt **92**. Additionally, detents or protrusions **118** project outwardly from outer circumferential surface **98** of forward skirt **92** at a side opposite hook **114**. Protrusions **118** may define a substantially chevron-shape with well defined edges **119** facing toward rearward skirt **94**.

As shown in FIGS. 2 and 6, shield **140** comprises a rearward end **144** and a forward end **146**.

Forward end **146** of shield **140** includes a slot or longitudinal opening **160** formed by sidewalls **162** that extend downwardly from top wall **163** and run substantially opposite of one another in parallel along the length of slot **160** towards forward end wall **164.** Slot **160** is slightly wider than needle **40**. Sidewalls **162** include bottom edges **165** that extend substantially parallel to one another and parallel to top wall **163**.

Two axially spaced cannula finger locks **167** are located at one of sidewalls **162** and are configured to secure the used needle. Each cannula finger lock **167** extends from a location on a first of the sidewalls **162** adjacent the bottom edge **165** thereof and projects angularly toward the opposed sidewall **162** and toward the top wall **163**. The projection of each cannula finger lock **167** from the respective sidewall **162** preferably exceeds half the distance between the respective sidewalls. The cannula finger locks **167** are deflectable by the needle when the needle enters slot **160.** Once the needle passes the end of each cannula finger lock **167,** the respective cannula finger lock moves back to its original position so that the needle is permanently trapped in slot **160** by cannula finger locks **167**.

Rearward end **144** of shield **140** defines a collar engaging area **166** that is a continuation of slot **160**. Collar engaging area **166** includes a rearward end **168**, a forward end **170**, a top finger guide area **172**, sidewalls **174** that extend downwardly from top finger guide area **172**, an underside area **176** dimensioned for surrounding collar **90**, and extending arms **180** to support hold hanger bar **182**. Sidewalls **174** are spaced apart by a major width adjacent rearward end **168**. The major width is selected to enable sidewalls **174** to slide across diametrically opposite side surfaces of forward skirt **92** of collar **90**. Sidewalls **174** converge, however, toward forward end **170** to define a minor distance therebetween substantially equal to the distance between sidewalls **162** at forward end **146** of shield **140**. Sidewalls **174** include bottom edges **177** that face away from top finger guide area **172**. As shown most clearly in FIG. 6, bottom edges **177** curve toward top finger guide area **172** at locations between rearward end **168** and forward end **170** of collar engaging area **166**.

The extreme rear ends of sidewalls **174** on collar engaging area **166** include rounded ears **194** that project toward one another from opposed inner surfaces **175** of sidewalls **174**. Rounded ears **194** are disposed to engage detents **118** on collar **90**. More particularly, each rounded ear **194** includes a distal surface **195**, a proximal surface **197** and a curved surface **198** extending between distal and proximal surfaces **195** and **197**. Distal surface **195** is aligned to sidewall **174** at an angle of approximately 60° and proximal surface **197** is aligned to sidewall **174** at an angle of approximately 45°. Curved surface **198** extends smoothly and convexly between distal and proximal surfaces **195** and **197**. Proximal surfaces **197** of rounded ears **194** will engage detents **118** to deflect sidewalls **174** slightly away from one another as shield **140** approaches the second position. The apex of curved surface **198** on each rounded ear **194** passes the respective detent **118** on collar **90**. As a result, sidewalls **174** begin to return resiliently toward an undeflected condition. The resilient return of sidewalls **174** and raked distal surface **195** of ears **194** causes sidewalls **174** to snap against detents **118**. This snapping action provides a clear audible and tactile indication of shielding and occurs substantially when the used needle is trapped by cannula finger locks **167**. The angles of distal and proximal surfaces **195** and **197** of rounded ears **194** affect the performance of shield **140**. In particular, a smaller acute angle alignment of proximal surface **197** reduces the force required to move shield **140** passed rounded ears **194**. A larger acute angle proximal surface **197** of rounded ears **194** requires a greater force to move shield **140** toward the second position. Similarly, the angle between distal surface **195** and sidewall **174** affects the acceleration characteristics as shield **140** is propelled toward the second position in response to the resilient return of sidewalls **174**. This change in acceleration characteristics affects the audible indication of shielding.

Top finger guide area **172** comprises a first ramp **184** that extends slightly on an upwardly slope from the rearward end of the collar engaging area to a shoulder **186**. From shoulder **186** extends a second ramp **188** which slopes downwardly towards top section **163**. Most preferably, first ramp **184** comprises touch bumps **190**. The touch bumps provide a tactile and visual guide to alert the user that the user's finger has contacted the shield and that the shield is in a defined or controlled position. The touch bumps may be any configuration so long as they extend and are distinct from the top finger guide area. The touch bumps may also be of a distinguishing color as compared to the top finger guide area or the shield.

Second ramp **188** has interior surface **192** for urging the needle toward the center of slot **160** as the shield is being rotated into the closed position. The exterior surfaces are slightly inclined and extending radially from the second ramp. The interior surfaces are especially helpful if the longitudinal axis of the needle is misaligned with respect to the longitudinal axis of the hub.

Extending arms **180** are located at rearward end **168** and at the beginning of top finger area **172** and hold hanger bar **182**.

The safety shield assembly and the needle assembly are assembled together whereby needle **40** is connected to hub **60** and sealed with adhesive at the ends of the hub. Hub **60** is then joined with collar **90** by ultra-sonic welding techniques or any other bonding techniques, or mechanical fit, whereby rearward annular skirt **94** of collar **90** mates with ribbed end **66** of the hub. Male ribs **82** of the hub are contained or forced fitted within inner sidewall **102** of rearward annular skirt **94** of collar **90**. The collar is aligned with the intravenous end of the needle whereby the hook arm is aligned with the bevel up of the needle. Then rigid sleeve **50** is force fitted into inner side wall **96** of forward skirt **92** of collar **90** to cover the needle. Thereafter, shield **140** is connected to collar **90** whereby hanger bar **182** is force fitted into hook member **114** whereby slot **160** faces rigid sleeve **50**. Most preferably, the shield is connected to the collar by a force fit or interference fit between the hanger bar and the hook bar. Therefore, the shield is always oriented in a stable position and will not move unless movement of the shield is positively initiated by the user. To assemble the last piece, shield **140** is moved towards rigid sleeve **50** and second rigid sleeve **52** is force fitted onto outer sidewall **104** of rearward skirt **94** of collar **90**.

In addition, a label **196** may be applied to the finally assembled parts. The label may be used to prevent tamper resistance of the parts, so that they are not reused.

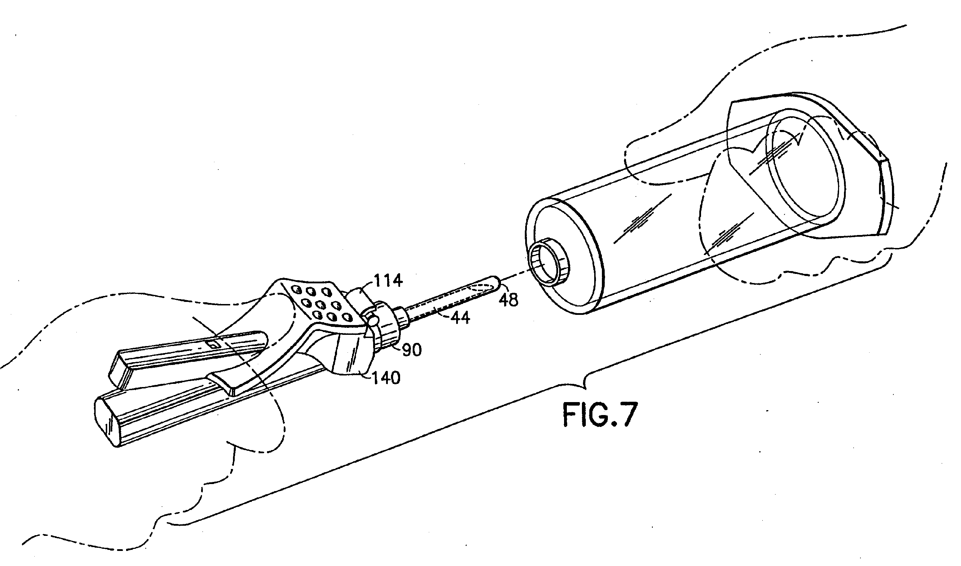

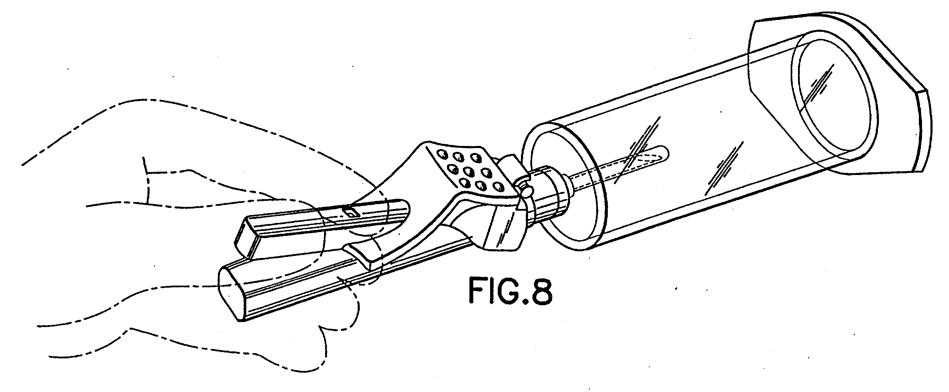

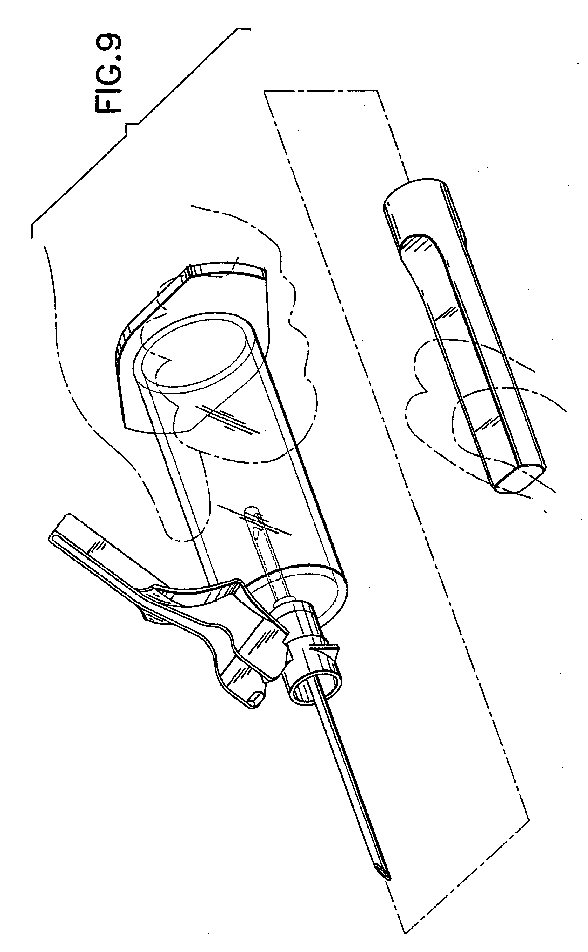

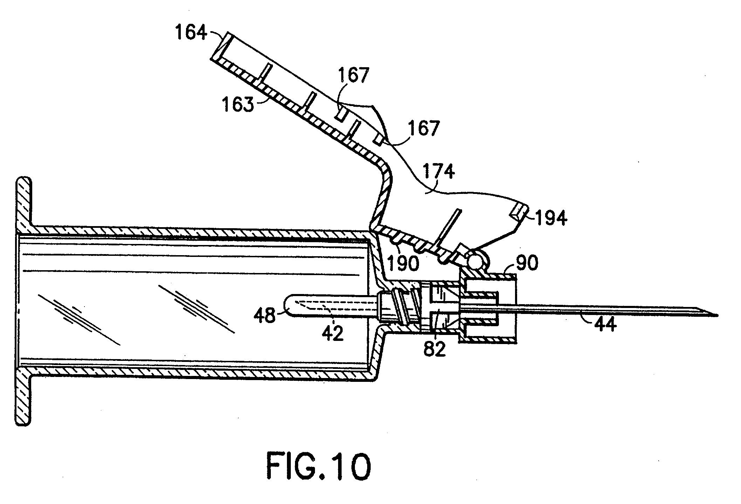

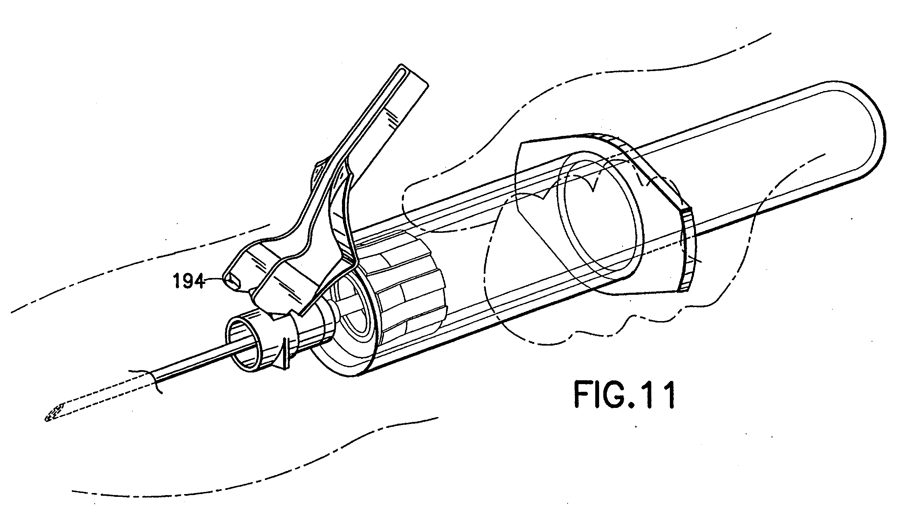

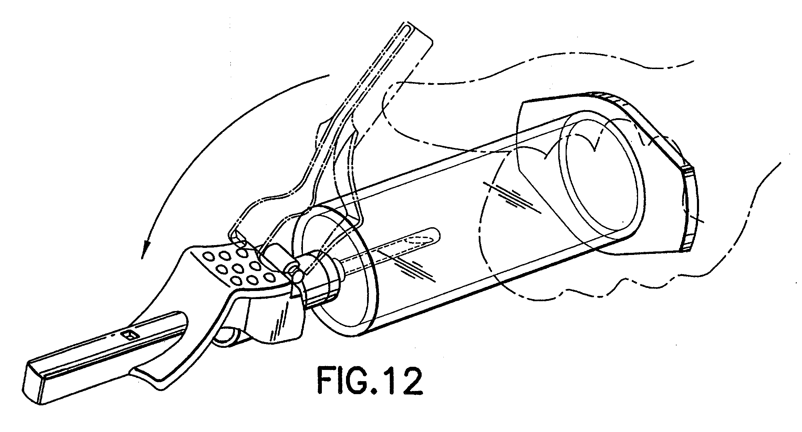

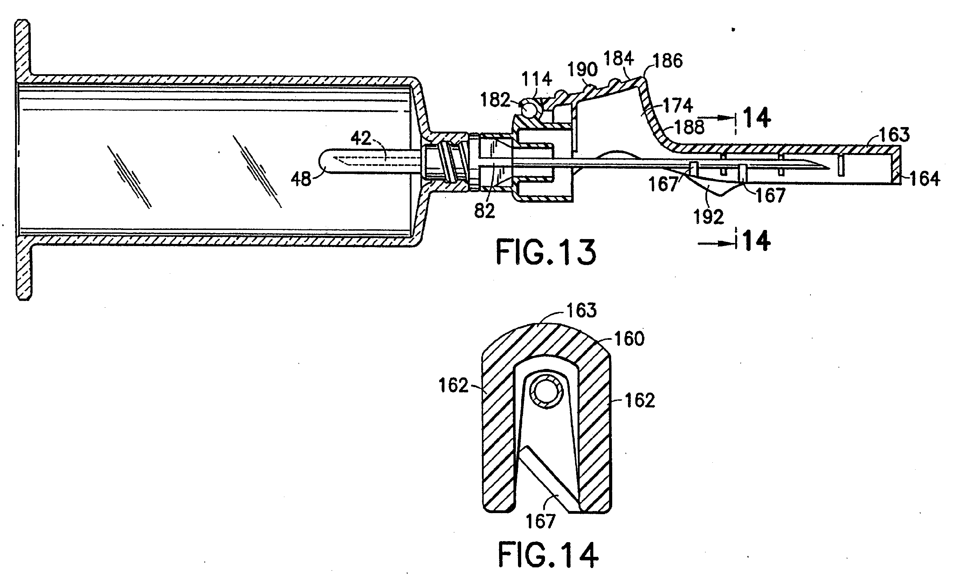

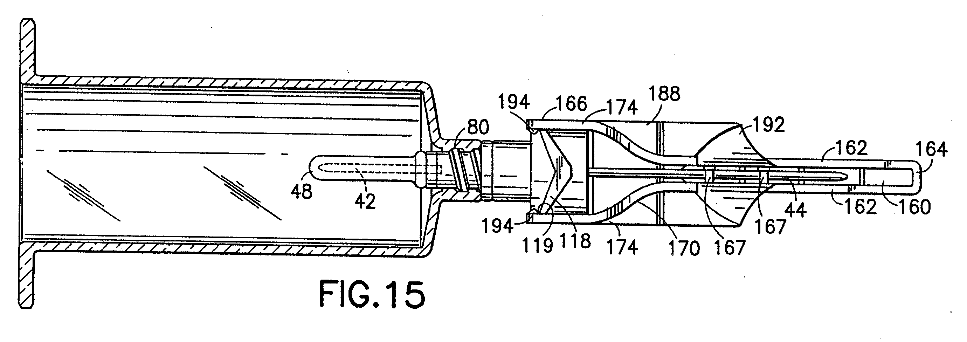

In use, as shown in FIGS. 7-15, the non-patient needle shield is removed and then a needle holder is screwed onto the hub of the needle. As specifically shown in FIGS. 8 and 12 the shield is then rotated back by the user towards the needle holder. Then as shown in FIG. 9, the intravenous needle shield is removed from covering the intravenous needle. Then as shown in FIG. 10, a venipuncture is conducted whereby the intravenous end of the needle is inserted into a vein of a patient and an evacuated tube having a closure is inserted into the needle holder. Then as shown in FIGS. 11 and 13, when the venipuncture is complete the user easily rotates the shield from the open position towards the intravenous needle to an intermediate position and then the user pushes on the shield at the top finger guide area to move the shield into a second position whereby the needle is trapped in the longitudinal opening. More particularly, needle **40** contacts cannula finger locks **167.** The engagement of needle **40** with cannula finger locks **167** causes each cannula finger lock **167** to deflect toward top wall and toward the sidewall **162** from which the respective cannula finger lock **167** projects. Sufficient rotation of shield **140** will cause needle **40** to pass both cannula finger locks **167**. As a result, cannula finger locks **167** will return resiliently to an undeflected condition. Thus, needle **40** will be trapped above cannula finger lock **167**.

Needle **40** is contained within shield **140** as the shield is pivoted into the second position. More particularly, proximal surfaces **197** of rounded ears **194** move over detents **118** and cause sidewalls **174** to deflect away from one another. The angularly aligned proximal faces **197** of rounded ears **194** ensures easy movement of shield **140**. Additionally, the resiliency of sidewalls **174** and the angular alignment of distal surface **195** of ears **194** causes shield **140** to be accelerated into the full second position. This accelerated movement of shield **140** helps to generate a clear audible and tactile indication of shielding.

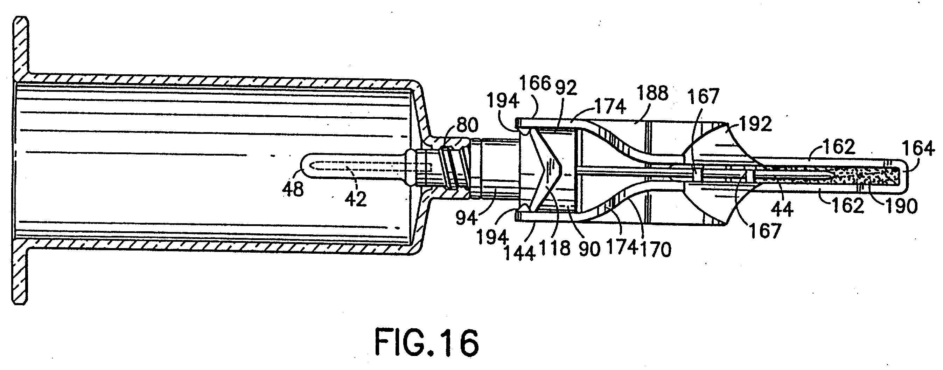

Alternatively as shown in FIG. 16, a gel material **190** is located in shield **140** so that when the needle snaps past cannula finger lock **167** it will come to rest in gel material **190**. The gel material will contain any residual fluid that may be on the needle. Simultaneously, rounded ears or projections **198** move over detents **118**. This causes sidewalls **174** to deflect away from one another and then to snap back into engagement with collar **90** to provide a clear audible and tactile indication of complete shielding.

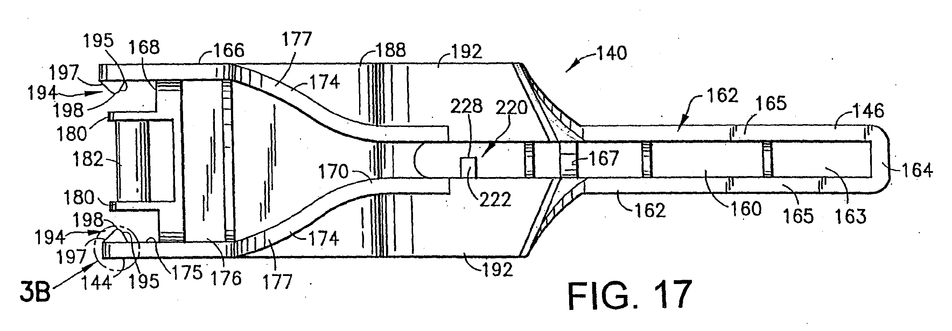

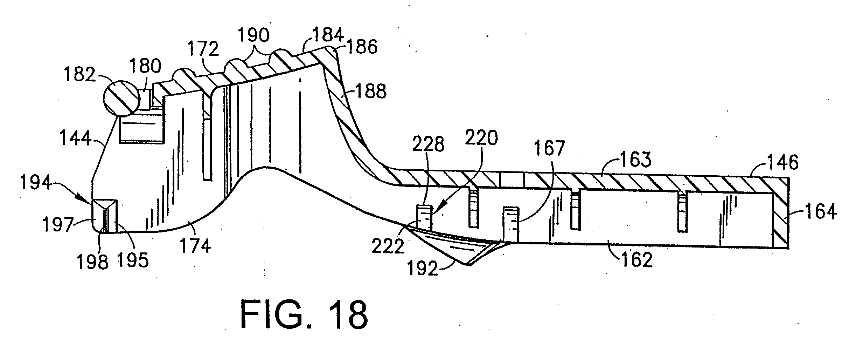

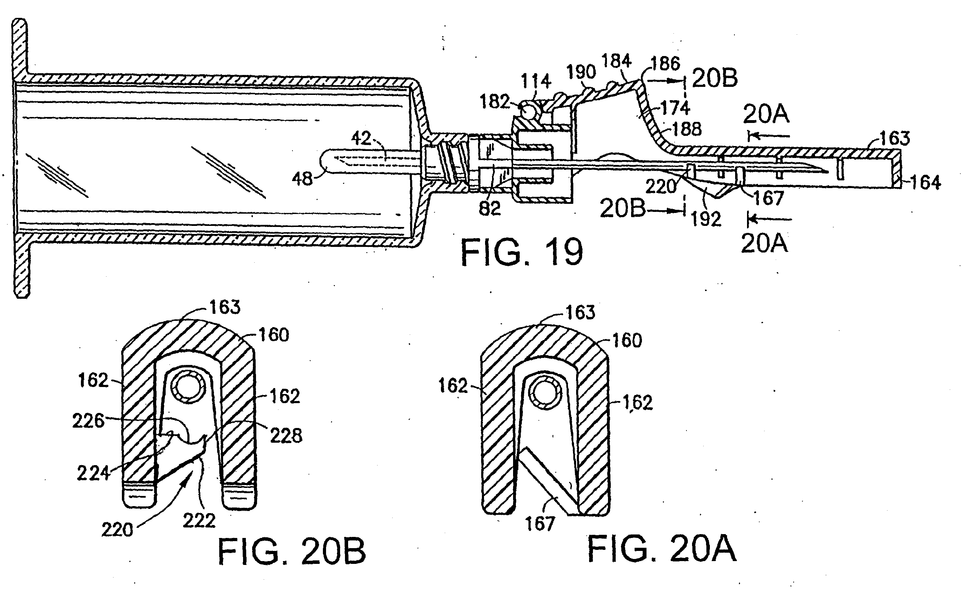

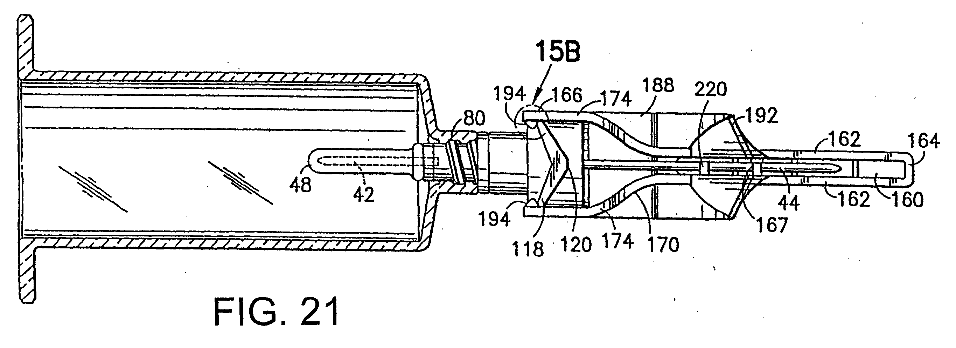

FIGS. 17-21 show a needle assembly that includes a needle **40**, a hub **60** and a collar **90** that are substantially identical to the embodiment of FIGS. 1-16. However, the needle assembly of FIGS. 17-21 includes a shield **140'** that differs from a shield **140** shown in the embodiment of FIGS. 1-16. Elements of the shield **140'** that are the same as or similar to elements in shield **140** are not described in detail herein, but rather are merely identified by the same reference numerals. Shield **140'** differs from shield **140** in that only a single cannula finger lock **167** is located at one of sidewalls **162** and is configured to secure the used needle. Cannula finger lock **167** extends from a location on a first of the sidewalls **162** adjacent the bottom edge **165** thereof and projects angularly toward the opposed sidewall **162** and toward the top wall **163**. The projection of the cannula finger lock **167** from the respective sidewall **162** preferably exceeds half the distance between the respective sidewalls. Cannula finger lock **167** is deflectable by the needle when the needle enters slot **160**. Once the needle passes the end of cannula finger lock **167**, the cannula finger lock moves back to its original position so that the needle is permanently trapped in slot **160** by cannula finger lock **167**.

Shield **140'** further includes a cannula shelf lock **220.** Cannula shelf lock **220** is a substantially planar and substantially rigid panel that projects orthogonally from one side wall **174** at a location at or near the interface of forward sidewalls **162** and rearward sidewalls **174**. Cannula shelf lock **220** includes a bottom edge extending substantially from bottom edge **177** of sidewall **174** angularly toward top wall **163** and/or top finger guide area **172.** Cannula shelf lock **220** further includes a top edge **224** aligned substantially parallel to the axis about which shield **140'** rotates. Top edge **224** includes a cylindrically generated concavity **226** generated about an axis extending parallel to top wall **163** and dimensioned to accommodate needle **44**. Slanted bottom edge **222** and top edge **224** meet at a corner **228** that is spaced from the opposed sidewall of shield **140'** by a distance that exceeds the outside diameter of needle **44.**

The needle assembly shown in FIGS. 17-21 is used substantially in the same manner as the needle assembly shown in FIGS. 1-16. When the venipuncture is complete the user easily rotates the shield from the open position towards the intravenous needle to an intermediate position and then the user pushes on the shield at the top finger guide area to move the shield into a second position whereby the needle is trapped in the longitudinal opening. More particularly, needle **44** contacts cannula finger lock **167** and cannula shelf lock **220**. The engagement of needle **44** with cannula finger lock **167** causes cannula finger lock **167** to deflect toward top wall and toward the sidewall **162** from which cannula finger lock **167** projects. Simultaneously, sloped bottom edge **222** of cannula shelf lock **220** will cause needle **44** to deflect. Sufficient rotation of shield **140'** will cause needle **44** to pass both cannula finger lock **167** and cannula shelf lock **220**. As a result, cannula finger lock **167** will return resiliently to an undeflected condition and needle **44** will return resiliently to an undeflected condition. Thus, needle **44** will be trapped above cannula finger lock **167** and above cannula shelf lock **220**. Additionally, needle **44** will be retained securely in concave region **226** of cannula shelf lock **220**. The combination of cannula finger lock **167** and cannula shelf lock **220** can provide more secure protection than a single cannula finger lock. More particularly, a cannula finger lock provides a secure trapping of needle **44**, albeit with relatively low resistance to a forced attempt to intentionally re-expose needle **44**. On the other hand, shelf lock **220** provides somewhat less effective trapping than cannula finger lock **167** in that a transverse shifting for the shield could bypass a cannula shelf lock that was used alone. However, a cannula shelf lock provides much more secure resistance to a forcible attempt to rotate shield **140'** back to its initial position. Thus, the cannula finger lock **167** and cannula shelf lock **220** cooperate to provide significantly enhanced trapping and resistance to re-exposure of cannula **44**.

Needle **44** is contained within shield **140'** as the shield is pivoted into the second position. More particularly, proximal surfaces **197** of rounded ears **194** move over rounded ends **124** of the chevron-shaped projection on collar **90** and cause sidewalls **174** to deflect away from one another. The angularly aligned proximal faces **197** of rounded ears **194** ensures easy movement of shield **140'**. Additionally, the resiliency of sidewalls **174** and the angular alignment of distal surface **195** of ears **194** causes shield **140'** to be accelerated into the second position. This accelerated movement of shield **140'** helps to generate a clear audible and tactile indication of shielding.

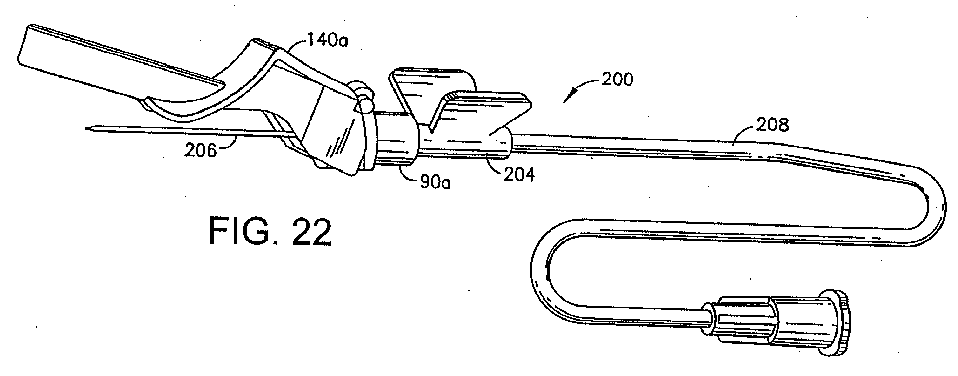

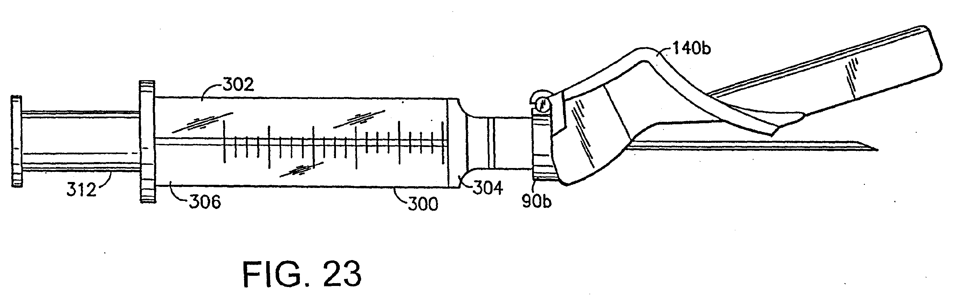

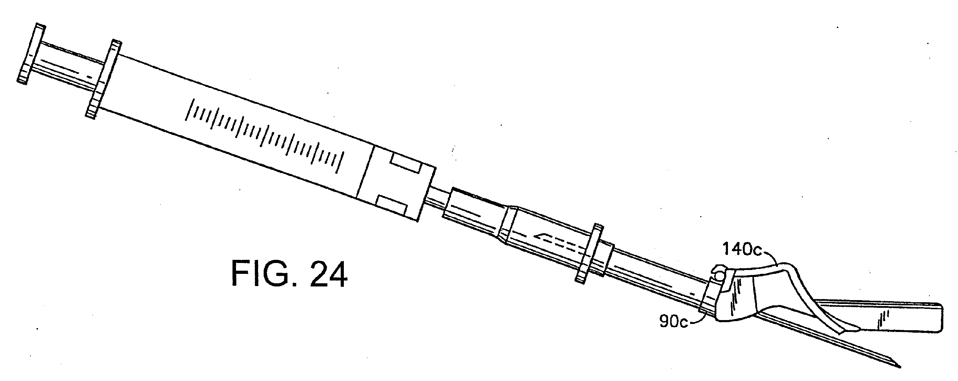

FIGS. 22, 23, and 24 are further embodiments of the invention that may include components which are substantially identical to the components of FIGS. 1-3. Accordingly, similar components performing similar functions will be numbered identically to those components of FIGS. 1-3, except that a suffix "a" will be used to identify those similar components in FIG. 22, a suffix "b" will be used to identify those similar components in FIG. 23 and a suffix "c" will be used to identify those similar components in FIG. 24.

Alternatively, the safety shield assembly of the present invention may be used in conjunction with a conventional intravenous (IV) infusion set, as illustrated in FIG. 22.

For purposes of illustration, shield **140a** and collar **90a** are connected to a conventional IV infusion set, **200**, or butterfly structure comprising a needle body with a needle hub **204** extending from the forward end of the needle body and a needle **206** embedded in hub **204**. Extending from the rearward end of the needle body is flexible tubing **208** which is conventional and utilized to allow the user to manipulate the structure and to connect it subsequently to supplies of infusion liquids or for the return of collected blood if the arrangement is being used to collect blood.

Infusion set **200** further comprises flexible wings **210** attached to and projecting outwardly from needle hub **204**.

Alternatively, the safety shield assembly of the present invention may be used in conjunction with a syringe, as illustrated in FIG. 23.

For purposes of illustration, shield **140b** and collar **90b** are connected to a conventional hypodermic syringe **300** comprising a syringe barrel **302** having a distal end **304** a proximal end **306** and a plunger **312.**

Alternatively, the present invention may be used in conjunction with a catheter as illustrated in FIG. 24.

The shield and collar of the safety shield assembly of the present invention are comprised of moldable parts which can be mass produced from a variety of materials including, for example, polyethylene, polyvinyl chloride, polystyrene or polyethylene and the like. Materials will be selected which will provide the proper covering and support for the structure of the invention in its use, but which will provide also a degree of resiliency for the purpose of providing the cooperative movement relative to the shield and the collar of the assembly.

## Claims

1. A safety needle assembly comprising a needle hub with proximal and distal ends and a passage extending between said ends, detents formed externally on said hub, a needle cannula mounted to said passage of said needle hub and having a pointed distal end projection beyond said distal end of said hub, a shield having proximal and distal ends, said proximal end of said shield being hingedly mounted to said hub for rotation from a first position where said shield is spaced from said needle cannula to a second position where said shield substantially surrounds said needle cannula, said shield comprising a top wall and opposed first and second sidewalls extending from said top wall, said sidewalls having bottom edges remote from said top wall, two cannula locks projecting from spaced apart locations on said first sidewall, rounded ears formed on said shield for engaging said detents when said shield is in said second position, whereby said cannula locks engage said needle cannula during rotation of said shield toward said second position such that at least one of said cannula locks and needle cannula deflect during said rotation for trapping said needle cannula, such that said needle cannula is trapped below said top wall and above said cannula locks, said rounded ears and said detents providing audible and tactile indication of said shield reaching said second position.

2. The safety needle assembly of claim 1, wherein at least one of said cannula locks is a resiliently deflectable cannula finger lock that projects angularly from said first sidewall toward said top wall.

3. The safety needle assembly of claim 1 or 2, wherein at least one of said cannula locks is a cannula shelf lock having a bottom edge sloped toward said top wall and a top edge spaced from said top wall, said cannula shelf lock generating deflection of said needle during said rotation of said shield to said second position such that said needle cannula is trapped below said top wall and said cannula shelf lock.

4. The safety needle assembly of claim 3, wherein the needle cannula defines an outside diameter, said cannula shelf lock being spaced from said second side wall of said shield by a distance greater than said outside diameter of said needle cannula.

5. The safety needle assembly of claim 3 or 4, wherein said top edge of said cannula shelf lock is substantially parallel to said top wall of said shield.

6. The safety needle assembly of any one of claims 3-5, wherein the top edge of said cannula shelf lock comprises a concave region facing said top wall for trapping said needle cannula, said concave region preferably defining a cylindrically generated edge aligned and configured for engaging an outer circumferential surface of said needle cannula.

7. The safety needle assembly of any one of claims 3-6, wherein said shield is hingedly mounted to said hub for rotation about an axis, said top edge of said cannula shelf lock being substantially parallel to said axis.

8. The safety needle assembly of any one of claims 3-7, wherein said cannula shelf lock is substantially planar and substantially orthogonal to said first sidewall.

9. The safety needle assembly of any one of claims 1-8, wherein said detents comprise two spaced apart detents disposed at locations on said hub substantially opposite said hinged connection of said shield to said hub.

10. The safety needle assembly of any one of claims 1-9, wherein said rounded ears are formed on inwardly facing surfaces of said sidewalls of said shield, and preferably are disposed.

11. The safety needle assembly of any one of claims 1-10, wherein said rounded ears are dimensioned to require deflection of said sidewalls away from one another as said shield is rotated into said second position and as said rounded ears move over said detents.

12. The safety needle assembly of any one of claims 1-11, wherein each said rounded ear comprises a proximal face aligned to said respective sidewall at an acute angle, a distal face aligned to said respective side wall at an acute angle and a curved surface extending between said proximal and distal faces, said distal face of each said ear preferably being aligned to said respective sidewall at an angle of approximately 60°.
